# EUROPEAN PATENT SPECIFICATION

(11) **EP 4 404 999 B1**
(45) Date of publication and mention of the grant of the patent: **08.07.2026**
(21) Application number: 22797302.1
(22) Date of filing: 25.09.2022
(51) Int. Cl.: A61M 5/168, G01N 1/14, G01N 35/00, G01N 35/10, B01L 3/02

(54) **NEEDLE WITH VENTING GROOVES**
NADEL MIT ENTLÜFTUNGSRINNEN
CANULE AVEC RAINURES DE VENTILATION

(30) Priority: 24.09.2021 EP 21198960
(43) Date of publication of application: 31.07.2024
(73) Proprietor: Accroma Labtec AG, 4132 Muttenz (CH)
(72) Inventor: SALADIN, Sven, 4153 Reinach (CH); GAFNER, Stefan, 4444 Rümlingen (CH)
(74) Representative: Latscha Schöllhorn Partner AG
(86) International application number: PCT/EP2022/076602
(87) International publication number: WO 2023/046938

(56) References cited:
- WO-A1-2019/038714
- US-A1- 2008 118 989

## Description

### Technical Field

The present invention relates in particular to a needle for delivering solutions and suspensions into and/or withdrawing them from a vessel closed by a septum and using rotary positive displacement pumps. Such a needle can be used for instance in analysis laboratories where relatively delicate, complex and costly machines are employed which are designed to perform sophisticate chemical or composition measurements, such as liquid chromatographers, titrators, water analyzers or similar.

In particular, high-performance liquid chromatography (HPLC, formerly referred to as high-pressure liquid chromatography), or ultra-performance liquid chromatography (UPLC) which are used to separate, identify, and quantify each component in a sample mixture, require that a fluid sample reaches a defined concentration as accurate as possible, so that the content of the substance to be analyzed can be determined as accurately as possible with the analytical machinery.

Sample materials processed can take many forms and can be inorganic, organic or biological. In particular, the sample material can be a hard and/or dry and/or brittle material, but also a softer material containing moisture, as in a biological tissue, or a viscous flowing mass in general, is conceivable. Therefore such needles may be employed not only in the chemical and bio-chemical industry, but for instance also in food industry, e.g. if a food sample is analyzed for heavy metals and similar.

### Background Art

Patent publication WO 2019/038714 A1 relates to the handling of liquids and gases, both extraction from and injection into, containers that can remain closed, if necessary, with the sampling needle of the disclosure being able to pierce through the top, bottom or side walls of the container.

According to conventional practice in sample preparation, a sample can be collected from a storing position, wherein the sample is typically contained in a vial retained in vial rack recess. The vial in which the sample is contained needs then to be carefully opened in order to enable taking out the sample and its consequent introduction into an extraction device.

Separation processes aimed at making a representative sample of a given material suitable for a subsequent chemical analysis require a preliminary fine size-reduction and homogenisation. Laboratory sample preparation protocols, in fact, require that the sample be manipulated, for instance by heating, screening, grinding, mixing etc., up to a target point of dissolution.

The extraction device can be operated to bring samples into a homogenous pulverized state and to prepare them perfectly for either additional sample preparation steps, and/or for subsequent analysis steps. Subsequent analysis steps can be separating analytical methods like high-performance liquid chromatography (HPLC), or ultra-performance liquid chromatography (UPLC), used to separate, identify, and quantify each component in a sample mixture.

As already mentioned, the target concentration of a fluid sample should be reached as accurate as possible, prior to submission to analysis machinery because of the adverse effect on the quantitation of the substance to be analysed.

Concentrations deviating from the specification can negatively influence the calculation and evaluation of the results output by the analysis machinery.

Conventional needles for the purpose of dispensing and/or withdrawing fluid samples comprise a needle tip and a single groove for venting which has been milled throughout. If a septum is pierced and dispensed into and/or extracted from a closed vessel, pressure equalisation should take place via this groove. With a single groove, however, there is a possibility, especially with thicker septa, that this groove will be blocked and thus the pressure equalisation will not work.

However, if this pressure equalisation does not take place, there is an increased counter pressure or under pressure which, in the case of rotary displacement pumps, results in irregular deviations when dosing and/or extracting predefined volumes greater than about 1 ml. Furthermore, with such conventional needles it is not possible to dispense a withdrawn sample liquid through a filter into a vessel, because the sample liquid cannot be dispensed through a filter due to the slit just mentioned, as in this case increased back pressure is required for it to flow through the filter medium.

Therefore, there is a need for a needle for use in automated system for the preparation of a sample for a chemical or composition analysis or such a system inside closed vessels allowing a highly efficient, automatized handling of a substantial number of samples and, at the same time, guaranteeing that the dispensed and/or withdrawn quantities are unaffected for the whole sample amount such that the analysis machines receive accurate measurable analytes. To ensure a high throughput of processed samples to be delivered to the analysers, the needle should at the same time be capable of cleanly processing a relatively large volume of fluid delivered and/or withdrawn through it at a compatible flow rate.

It is therefore the object of the present invention to provide a needle by means of which the above-mentioned shortcomings may overcome.

### Disclosure of the Invention

According to the invention these needs are settled by a needle for use in an automated system for the preparation of a fluid sample for a chemical or composition analysis. The needle comprises a needle shaft which is followed at its distal end by a needle tip portion configured for dispensing or withdrawing the fluid sample. The needle shaft comprises at its proximal end a connection section configured for providing a connection with the automated preparation system. The needle tip portion comprises a transition section and a needle tip, wherein the transition section connects the needle shaft with the needle tip. The needle shaft comprises at least two grooves.

According to the invention, the at least two grooves are configured to provide for pressure equalization when dispensing or withdrawing fluid samples with a larger volume. A "larger volume" as defined herein is a volume of more than 1 ml.

Preferably, the needle shaft comprises a cylindrical shape. This shape has proven to be the most effective.

According to the invention, the at least two grooves are in the form of longitudinal cutouts extending over the needle shaft up to (but not into) in the distal direction, the transition section of the needle tip portion. In other words, the grooves are not milled all the way into the tip portion but end at the distal end of the shaft, i.e. in close vicinity of the transition section.

Preferably, the transition section comprises a conical shape. Hereby, a very efficient dispensing or withdrawing of fluid sample is ensured.

Preferably, the at least two grooves are parallel to each other. This has proven to be advantageous with regard to the required pressure equalization.

Preferably, the at least two grooves are evenly distributed along the circumference of the needle shaft. Hereby, the required pressure equalization is further supported.

Preferably, in a top view, one of the at least two grooves is flush with the bevel of the needle tip. Also this measure contributes to further improved pressure equalization.

Preferably, the grooves include, in case of two, three, four, five, six, seven or eight grooves, a circumferential angle of at least 45°. Hereby, a particularly efficient design is achieved.

In a further aspect, the invention includes a method for the preparation of a fluid sample for a chemical or composition analysis with an inventive needle providing pressure equalization when dispensing or withdrawing a fluid sample with a larger volume, preferably with a volume of more than 1 ml.

In a still further aspect, the invention includes an automated system for the preparation of a fluid sample for a chemical or composition analysis, the system comprising an inventive needle and an injection head for the injection of an amount of sample in a receptacle through a filter apparatus.

Preferably, the at least two grooves of the inventive needle are configured to provide preventing pressure equalization when injecting the sample or an amount of the sample in the receptacle and in the filter apparatus.

The automated system for the preparation of a sample for a chemical or composition analysis preferably comprises an injection head for the injection of an amount of sample in a receptacle through a filter apparatus into a vessel and via an inventive needle.

The method is preferably used for automated preparation of a sample for chemical or compositional analysis using the automated system.

### Brief Description of the Drawings

The needle according to the present invention is described in more detail herein below by way of exemplary embodiments and with reference to the attached drawings, in which:
- Fig. 1: shows a prior art needle for dispensing or withdrawing a sample solution or suspension; and
- Fig. 2: shows a needle in accordance with the present invention.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

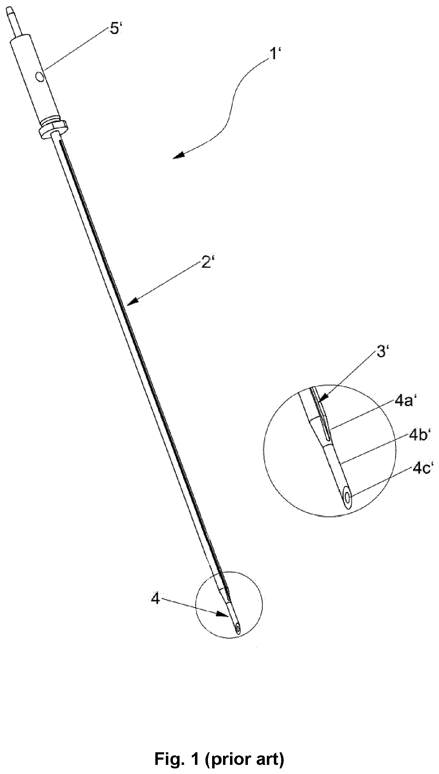

In **Fig. 1****,** a known needle 1' is illustrated. The needle 1' has a needle shaft 2' with a connection section 5' at its proximal end and with a single longitudinal groove 3' therein. The longitudinal groove 3' extends along the needle shaft 2' and into the transition section 4a', i.e. up to or almost up to the needle tip 4b'. At the distal end, the bevel of the needle tip is designated 4c'.

Here, the single groove which is used for venting has been milled throughout, i.e. up to the needle tip. If a septum is pierced and dispensed into and/or extracted from a closed vessel, pressure equalization should take place via this groove. With a single groove, however, there is a possibility, especially with thicker septa, that this groove will be blocked and thus the pressure equalisation will not work. If this pressure equalisation does not take place, there is an increased counter pressure or under pressure which, in the case of rotary displacement pumps, results in irregular deviations in particular when dosing or extracting predefined volumes greater than 1 ml. Furthermore, such grooves that are milled throughout allow pressure equalization when using a filter apparatus where such pressure equalization is however not desired.

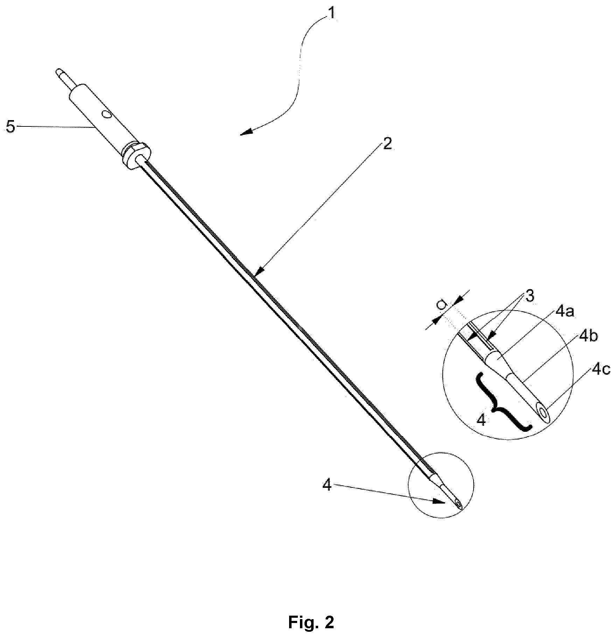

In **Fig. 2****,** a needle 1 in accordance with the present invention is shown. At its proximal end the needle 1 comprises a connection section 5 from which the needle shaft 2 extends up to the tip portion 4 and in particular up to the transition section 4a. The tip portion 4 comprises the transition section 4a, the needle tip 4b and the bevel of the needle tip 4c. As one can see, two grooves are not milled throughout, i.e. the grooves 3 end within the needle shaft 2 adjacent the transition section 4a. Further, in the top view, the uppermost respectively right groove is flush with the needle bevel 4c. Preferably the two or more grooves are arranged parallel to one another and are evenly distributed about the circumference of the needle shaft 2. Reference sign α designates the circumferential angle included between two grooves 3. In case of two, three, four, five, six, seven or eight grooves 3, the circumferential angle α is at least 45°.

The inventive needle 1 allows for efficient pressure compensation and thereby enables volumes larger than 1 ml to be dispensed constantly and accurately with a rotary displacement pump into a vessel closed by a septum or to be extracted therefrom. At the same time, it is possible to dispense an extracted sample fluid through a filter apparatus without pressure compensation, as otherwise the fluid will not pass through the filter medium of the filter apparatus.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the figures individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

### List of reference numbers:

- 1': prior art needle
- 2': needle shaft
- 3': groove
- 4': needle tip portion
- 4a': transition section
- 4b': needle tip
- 4c': bevel of needle tip
- 5': connection section
- 1: inventive needle
- 2: needle shaft
- 3: grooves
- 4: needle tip portion
- 4a: transition section
- 4b: needle tip
- 4c: bevel of needle tip
- 5: connection section
- α: circumferential angle between two grooves

## Claims

1. A needle (1) for use in an automated system for the preparation of a fluid sample for a chemical or composition analysis, the needle (1) comprising:
a needle shaft (2) which is followed at its distal end by a needle tip portion (4) configured for dispensing or withdrawing the fluid sample, wherein
the needle shaft (2) comprises at its proximal end a connection section (5) configured for providing a connection with the automated preparation system, wherein
the needle tip portion (4) comprises a transition section (4a) and a needle tip (4b), wherein the transition section (4a) connects the needle shaft (2) with the needle tip (4b) and wherein the needle shaft (2) comprises at least two grooves (3), **characterized in that**,
the at least two grooves (3) are configured to provide for pressure equalization when dispensing or withdrawing fluid samples with a volume of more than 1 ml wherein the at least two grooves (3) are in the form of longitudinal cutouts extending over the needle shaft (2) up to, in the distal direction, the transition section (4a) of the needle tip portion (4).

2. The needle (1) according to claim 1, wherein the needle shaft (2) comprises a cylindrical shape.

3. The needle (1) according to any one of the preceding claims, wherein the transition section (4a) comprises a conical shape.

4. The needle (1) according to any one of the preceding claims, wherein the at least two grooves (3) are parallel to each other.

5. The needle (1) according to any one of the preceding claims, wherein the at least two grooves (3) are evenly distributed along the circumference of the needle shaft (2).

6. The needle (1) according to any one of the preceding claims, wherein, in a top view, one of the at least two grooves (3) is flush with the bevel of the needle tip (4b).

7. The needle (1) according to any one of the preceding claims, wherein, in case of two, three, four, five, six, seven or eight grooves (3), the grooves (3) include a circumferential angle α of at least 45°.

8. Method for the preparation of a fluid sample for a chemical or composition analysis with a needle (1) according to any one of claims 1 to 7, **characterized by** providing pressure equalization when dispensing or withdrawing a fluid sample with a volume of more than 1 ml.

9. Automated system for the preparation of a fluid sample for a chemical or composition analysis, the system comprising a needle (1) according to any one of claims 1 to 7, and an injection head for the injection of an amount of sample in a receptacle through a filter apparatus.

10. Automated system for the preparation of a fluid sample for a chemical or composition analysis according to claim 9, wherein the at least two grooves (3) are configured to provide preventing pressure equalization when injecting the amount of the sample in the receptacle and in the filter apparatus.

## Patentansprüche

1. Eine Nadel (1) zur Verwendung in einem automatisierten System zur Vorbereitung einer Flüssigkeitsprobe für eine chemische Analyse oder für eine Zusammensetzungsanalyse, wobei die Nadel (1) umfasst:
einen Nadelschaft (2), an dessen distalem Ende sich ein Nadelspitzenabschnitt (4) anschließt, der zum Abgeben oder Entnehmen der Flüssigkeitsprobe ausgebildet ist, wobei
der Nadelschaft (2) an seinem proximalen Ende einen Verbindungsabschnitt (5) aufweist, der zur Herstellung einer Verbindung mit dem automatisierten Aufbereitungssystem ausgebildet ist, wobei
der Nadelspitzenabschnitt (4) einen Übergangsabschnitt (4a) und eine Nadelspitze (4b) umfasst, wobei der Übergangsabschnitt (4a) den Nadelschaft (2) mit der Nadelspitze (4b) verbindet und wobei der Nadelschaft (2) mindestens zwei Nuten (3) umfasst, **dadurch gekennzeichnet, dass**
die mindestens zwei Nuten (3) so ausgebildet sind, dass sie beim Abgeben oder Entnehmen von Flüssigkeitsproben mit einem Volumen von mehr als 1 ml für einen Druckausgleich sorgen, wobei die mindestens zwei Nuten (3) die Form von Längsausschnitten haben, die sich über den Nadelschaft (2), in distaler Richtung, bis zum Übergangsabschnitt (4a) des Nadelspitzenabschnitts (4) erstrecken.

2. Die Nadel (1) nach Anspruch 1, wobei der Nadelschaft (2) eine zylindrische Form aufweist.

3. Die Nadel (1) nach einem der vorstehenden Ansprüche, wobei der Übergangsabschnitt (4a) eine konische Form aufweist.

4. Die Nadel (1) nach einem der vorstehenden Ansprüche, wobei die mindestens zwei Nuten (3) parallel zueinander verlaufen.

5. Die Nadel (1) nach einem der vorstehenden Ansprüche, wobei die mindestens zwei Nuten (3) gleichmäßig entlang des Umfangs des Nadelschafts (2) verteilt sind.

6. Die Nadel (1) nach einem der vorstehenden Ansprüche, wobei in der Draufsicht eine der mindestens zwei Nuten (3) bündig mit der Abschrägung der Nadelspitze (4b) abschließt.

7. Die Nadel (1) nach einem der vorstehenden Ansprüche, wobei im Falle von zwei, drei, vier, fünf, sechs, sieben oder acht Nuten (3), die Nuten (3) einen Umfangswinkel α von mindestens 45° aufweisen.

8. Verfahren zur Vorbereitung einer Flüssigkeitsprobe für eine chemische Analyse oder für eine Zusammensetzungsanalyse mit einer Nadel (1) gemäß einem der Ansprüche 1 bis 7, **dadurch gekennzeichnet, dass** beim Abgeben oder Entnehmen einer Flüssigkeitsprobe mit einem Volumen von mehr als 1 ml ein Druckausgleich hergestellt wird.

9. Automatisiertes System zur Vorbereitung einer Flüssigkeitsprobe für eine chemische Analyse oder für eine Zusammensetzungsanalyse, wobei das System eine Nadel (1) gemäß einem der Ansprüche 1 bis 7 und einen Injektionskopf zum Injizieren einer Probenmenge in einen Behälter durch eine Filtervorrichtung umfasst.

10. Automatisiertes System zur Vorbereitung einer Flüssigkeitsprobe für eine chemische Analyse oder für eine Zusammensetzungsanalyse gemäß Anspruch 9, wobei die mindestens zwei Nuten (3) so ausgebildet sind, dass sie beim Injizieren der Probenmenge in den Behälter und in die Filtervorrichtung einen Druckausgleich verhindern.

## Revendications

1. Une aiguille (1) destinée à être utilisée dans un système automatisé de préparation d'un échantillon de fluide en vue d'une analyse chimique ou d'une analyse de composition, l'aiguille (1) comprenant :
une tige d'aiguille (2) suivie, à son extrémité distale, d'une partie formant pointe d'aiguille (4) configurée pour distribuer ou prélever l'échantillon de fluide, dans laquelle
la tige d'aiguille (2) comprend, à son extrémité proximale, une section de raccordement (5) configurée pour assurer une connexion avec le système de préparation automatisé, dans laquelle
la partie d'embout d'aiguille (4) comprend une section de transition (4a) et un embout d'aiguille (4b), dans laquelle la section de transition (4a) relie la tige d'aiguille (2) à l'embout d'aiguille (4b) et dans laquelle la tige d'aiguille (2) comprend au moins deux rainures (3), **caractérisé en ce que**,
les au moins deux rainures (3) sont configurées pour assurer l'égalisation de la pression lors de la distribution ou du prélèvement d'échantillons de fluide d'un volume supérieur à 1 ml, les au moins deux rainures (3) se présentant sous la forme d'encoches longitudinales s'étendant sur la tige d'aiguille (2) jusqu'à, dans la direction distale, la section de transition (4a) de la partie d'embout d'aiguille (4).

2. L'aiguille (1) selon la revendication 1, dans laquelle la tige d'aiguille (2) présente une forme cylindrique.

3. L'aiguille (1) selon l'une quelconque des revendications précédentes, dans laquelle la section de transition (4a) présente une forme conique.

4. L'aiguille (1) selon l'une quelconque des revendications précédentes, dans laquelle les au moins deux rainures (3) sont parallèles les unes aux autres.

5. L'aiguille (1) selon l'une quelconque des revendications précédentes, dans laquelle les au moins deux rainures (3) sont réparties uniformément le long de la circonférence de la tige d'aiguille (2).

6. L'aiguille (1) selon l'une quelconque des revendications précédentes, dans laquelle, en vue de dessus, l'une des au moins deux rainures (3) affleure le biseau de la pointe de l'aiguille (4b).

7. L'aiguille (1) selon l'une quelconque des revendications précédentes, dans laquelle, dans le cas de deux, trois, quatre, cinq, six, sept ou huit rainures (3), les rainures (3) forment un angle circonférentiel α d'au moins 45°.

8. Procédé de préparation d'un échantillon de fluide en vue d'une analyse chimique ou d'une analyse de composition à l'aide d'une aiguille (1) selon l'une quelconque des revendications 1 à 7, **caractérisé en ce qu'**il comprend une égalisation de pression lors de l'injection ou du prélèvement d'un échantillon de fluide d'un volume supérieur à 1 ml.

9. Système automatisé pour la préparation d'un échantillon de fluide en vue d'une analyse chimique ou d'une analyse de composition, le système comprenant une aiguille (1) selon l'une quelconque des revendications 1 à 7, et une tête d'injection pour l'injection d'une quantité d'échantillon dans un récipient à travers un dispositif de filtration.

10. Système automatisé pour la préparation d'un échantillon de fluide en vue d'une analyse chimique ou d'une analyse de composition selon la revendication 9, dans lequel les au moins deux rainures (3) sont configurées pour empêcher l'égalisation de pression lors de l'injection de la quantité d'échantillon dans le récipient et dans le dispositif de filtration.
